(19) Europäisches Patentamt
European Patent Office
Office européen des brevets

(11) Publication number: **0 010 320**
**B1**

(12) # EUROPEAN PATENT SPECIFICATION

(45) Date of publication of patent specification: **02.06.82**

(51) Int. Cl.³: **C 07 J 41/00,**
A 61 K 31/565,
A 61 K 31/575

(21) Application number: **79200531.6**

(22) Date of filing: **24.09.79**

(54) **Novel 3-beta-amino-2-beta-hydroxy steroids of the androstane, oestrane and pregnane series, processes for their preparation and pharmaceutical compositions.**

(30) Priority: **03.10.78 GB 3912778**

(43) Date of publication of application:
**30.04.80 Bulletin 80/9**

(45) Publication of the grant of the patent:
**02.06.82 Bulletin 82/22**

(84) Designated Contracting States:
**AT BE CH DE FR GB IT LU NL SE**

(56) References cited:
**GB - A - 1 108 563**
**GB - A - 1 439 605**

**BULLETIN DE LA SOCIETE CHIMIQUE DE FRANCE, 1964**
**Paris (FR)**
**M. JANOT et al. "No. 349 — Alcaloides stéroidiques, XXXI. Alcaloides des feuilles de l'Holarrhena antidysenteria (Roxb.) Wall. Structure de la kurchiline; synthèse des quatre hydroxy-2 diméthylamino-3 prégnane-5x diastéréoisomères". pages 2158—2166.**

(73) Proprietor: **Akzo N.V.**
**Velperweg 76**
**NL-6824 BM Arnhem (NL)**

(72) Inventor: **Boyd, Andrew Craig**
**14 Iona Ridge**
**Hamilton - Scotland (GB)**
Inventor: **Sleigh, Thomas**
**3 Coulter Avenue**
**Wishaw - Scotland (GB)**
Inventor: **Savage, David Samuel**
**32 Beech Avenue Newton Mearns**
**Glasgow - Scotland (GB)**

(74) Representative: **van 't Holt, Hendrik et al.**
**Postbus 20**
**NL-5340 BH Oss (NL)**

EP 0 010 320 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Novel 3β-amino-2β-hydroxy steroids of the androstane, oestrane and pregnane series, processes for their preparation and pharmaceutical compositions

This invention relates to novel 3β-amino-2β-hydroxy steroids of the androstane, oestrane and pregnane series and derivatives thereof, to processes for their preparation and to pharmaceutical compositions containing these compounds as active component.

In British Specification 1 108 563 amino steroids of the androstane, oestrane and pregnane series are described, having a hydroxyl or acyloxy group in 2β-position and a tertiary amino group in 3α-position. Some of these compounds have been found to possess anti-arrhythmic activity. However, at therapeutic dose levels these compounds also exhibit undesirable activities, such as convulsant activity and local anaesthetic activity which precludes their clinical application.

In British Specification 1 439 605 amino steroids of the androstane, oestrane and pregnane series are described, having a hydroxyl or acyloxy group in 2β-position and a primary amino group in 3α-position. These compounds have anti-arrhythmic properties and are virtually devoid of convulsant and local anaesthetic activities.

Surprisingly, it was found that novel steroids of the androstane, oestrane and pregnane series, substituted in 2β-position with a hydroxyl or acyloxy group, and in the 3β-position with a primary or secondary amino group, are potent anti-arrhythmic agents.

Therefore, the present invention relates to novel steroids of the androstane, oestrane and pregnane series having the formula I:

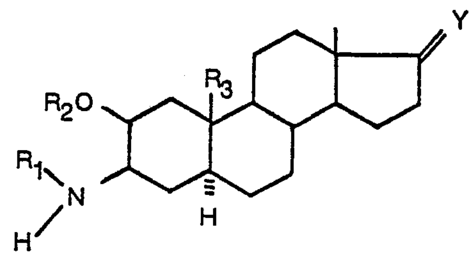

I

and pharmaceutically acceptable non-toxic addition salts thereof, wherein:

(a) $R_1$ is H, alkyl of one to six colours (preferably lower alkyl, such as methyl), or acyl of one to eighteen carbons;
(b) $R_2$ is H or acyl of one to eighteen carbons;
(c) $R_3$ is H or methyl, preferably methyl;
(d) Y is selected from the group consisting of O, acetalized O, H(β$OR_4$), $R_5$(β$OR_4$), H(β$COCH_3$) and H(β$CHOR_4CH_3$);
(e) $R_4$ is H or acyl of one to eighteen carbons; and
(f) $R_5$ is a saturated or unsaturated alkyl group of one to four carbons,

as well as compositions containing a pharmaceutically effective amount of one or more compounds of formula I.

A special group of compounds is the group having the formula II:

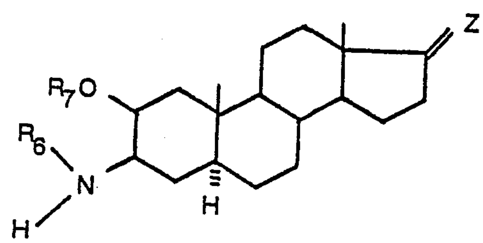

II

wherein:

(a) $R_6$ is H or methyl;
(b) $R_7$ is H or lower alkanoyl of one to four carbons; and
(c) Z is O, acetalized O, or H(β$OR_8$), wherein $R_8$ = H or lower alkanoyl of one to four carbons;

and acid addition salts and N-acetyl derivatives of these compounds. Preferably, $R_7$ is H and Z is O.

As stated above, the novel compounds have antiarrhythmic properties, have no or minimal and transient haemodynamic effects, have no local anaesthetic activity and do not cause CNS-stimulation. They also have prophylactic effect and decrease infarctsize.

The compounds according to the invention can be prepared by methods employing steps known to those in the art.

For example, one may start from the corresponding 3α-azido-2β-hydroxy compound. The 3α-azido-2β-hydroxy compound is first oxidized to give the corresponding 2-oxo-3α-azide, for example

with $CrO_3$, preferably in the form of the complex piridinium chlorochromate in buffered solution, then the 3α-azido group is epimerized to the 3β-azido group on silica gel, whereafter the groups present in 2- and 3-position are reduced with a complex metal hydride to give the desired 3β-amino-2β-hydroxy compound. The reduction is best carried out in two steps, first using an alkali metal borohydride, for example sodium borohydride, to give the 3β-azido-2β-hydroxy compound, followed by lithium aluminium hydride reduction of the azido group to give the 3β-amino-2β-hydroxy compound.

The starting 3α-azido-2β-hydroxy compound can be prepared by reacting a corresponding $\Delta^2$-steroid of the androstane, oestrane or pregnane series with a hypohalous acid, prepared in situ from an organic N-halo compound, e.g. N-bromo acetamide or N-chloro succinimide, and aqueous perchloric acid, to give the corresponding 3α-halo-2β-hydroxy compound, treating the 3α-halo-2β-hydroxy compound with alkali to give the corresponding 2β,3β-oxido steroid and reacting the 2β,3β-oxido compound with an alkali metal azide, for example sodium azide, to give the starting 3α-azido-2β-hydroxy steroid. (See British Patent Specification No. 1 439 605).

When the starting 3α-azido-2β-hydroxy steroid has an oxo group in 17- or 20-position, this oxo group is preferably protected during the reaction sequence for obtaining the 3β-amino-2β-hydroxy compound, e.g. in the form of its acetal such as the ethylene diacetal. After the introduction of the 3β-amino-2β-hydroxy group the compound is deacetalized in 17- or 20-position in the usual way, if required.

The 2β-hydroxy-3β-methyl-amino compound can readily be prepared from the 2β-hydroxy-3β-primary amino compound by N-formylation (heating with formic acid) followed by reduction of the N-formyl group, e.g. with a complex metal hydride such as lithium aluminiumhydride. A vulnerable oxo group, if present, is temporarily protected (e.g. in the form of its acetal) during the reduction step.

Another route to the 3β-amino-2β-hydroxy steroids consists in reacting a $\Delta^2$-steroid with a peracid e.g. peracetic acid, preferably in a two-phase system of chloroform and aqueous peracetic acid, to give the 2α,3α-peroxide, converting the 2α,3α-peroxide with benzyl alcohol in the presence of $BF_3$-etherate into the 2β-benzyloxy-3α-ol, reacting the 2β-benzyloxy-3α-ol with p-toluene sulphonic acid or methyl sulphonic acid so as to obtain the corresponding 2β-benzyloxy-3α-sulphonate, converting the sulphonate with sodium azide, preferably in N-methylpyrrolidone, into the 2β-benzyloxy-3β-azide, whereafter the azide is reduced with a complex metal hydride, e.g. lithiumaluminiumhydride, to the 3β-amino-2β-benzyloxy steroid. Hydrogenolysis of the benzyloxy group with Pd/C or $PtO_2$ in ethanol or acetic acid or with ferric chloride and acetic acid anhydride or with aqueous HBr under reflux gives the corresponding 3β-amino-2β-hydroxy steroid.

The conversion of the 3β-amino compound into acid addition salts thereof can be carried out by treatment with an inorganic acid such as hydrochloric acid, hydrobromic acid, phosphoric acid, or an organic acid, such as citric acid, pyruvic acid, succinic acid and fumaric acid.

A 3β-amino-2β-hydroxy steroid or a 3β-amino-2β-acyloxy steroid may be converted into the mono-N-acyl derivative by acylation with an acid anhydride in a suitable solvent such as methylene chloride. The resulting 3β-N-acyl-amino-2β-hydroxy steroid may be further converted into the 3β-N-acyl-amino-2β-acyloxy steroid, e.g. by treatment with an acid anhydride in pyridine.

It is self-evident that the N-acyl group may be different from the acyl group in 2β-position.

A hydroxy group in 17- or 20-position, if present, may be oxidized to the corresponding oxo group by known methods, for example, chromic acid in the presence of sulphuric acid.

A 17-oxo group, if present, may be reduced to a 17β-hydroxy group e.g. with $NaBH_4$, or alkylated to give the 17α-$R_5$-17β-OH compound.

Free hydroxy groups, if present in the molecule, may be acylated. The acylation may be carried out with an organic carboxylic acid, preferably with an aliphatic, cyclo-aliphatic, aromatic or araliphatic carboxylic acid with 1—18 carbon atoms, such as acetic acid, propionic acid, pentanoic acid, trimethylacetic acid, heptanoic acid, decanoic acid, dodecanoic acid, benzoic acid, β-phenyl propionic acid, cyclooctyl acetic acid, succinic acid, or with a functional derivative thereof, such as the anhydride or the acid chloride.

The new compounds according to the invention may be used in the form of pharmaceutical compositions, for which purpose they are mixed with one or more pharmaceutically acceptable non-toxic carriers and/or the usual excipients suitable for oral administration or for injection.

The effective oral dose is in the range from 0.5—25 mg/kg and the effective intravenous dose is in the range from 0.1—10 mg/kg.

The following examples illustrate the invention.

Example I

a) 3α-azido-2β-hydroxy-5α-androstan-17-one-ethylenedioxy ketal.

To 3α-azido-2β-hydroxy-5α-androstan-17-one (93.5 g) was added ethylene glycol (93.5 ml), triethylorthoformate (187.0 ml) and paratoluenesulphonic acid (5.8 g) and the solution refluxed under nitrogen for 1 hour. The mixture was now cooled to room temperature and poured into sodium carbonate solution. The precipitated product was filtered off, washed with water and dried. Crystallisation from ether afforded pure 3α-azido-2β-hydroxy-5α-androstan-17-one-ethylenedioxy ketal (95.76 g; 90.4%) as needles, m.p. 159—160°C, $[\alpha]_D$+46.7°; $CHCl_3$; C% 0.878.

b) $3\alpha$-azido-$5\alpha$-androstane-2,17-dione-17-ethylenedioxy ketal.

$3\alpha$-azido-$2\beta$-hydroxy-$5\alpha$-androstan-17-one-ethylenedioxy ketal (95.5 g) was dissolved in methylene dichloride (700 ml) and added in one portion to a stirred mixture of pyridinium chlorochromate (109.7 g) and anhydrous sodium acetate (62.0 g) in methylene dichloride (700 ml). The mixture was stirred for 2 hours, at room temperature and sodium dried ether (700 ml) now added. The resulting mixture was filtered through dicalite, to remove inorganics, and the liquors concentrated under reduced pressure. Ether (700 ml) was added and again the insoluble material filtered through dicalite. The solution was now filtered through a short silica column to remove colour and the liquors evaporated under reduced pressure to give an off-white solid. Crystallisation from ether afforded pure $3\alpha$-azido-$5\alpha$-androstane-2,17-dione-17-ethylenedioxy ketal (55.6 g), as prisms, m.p. 132—135°C, $[\alpha]_D$+8.39°; $CHCl_3$; C% 1.073.

c) $3\beta$-azido-2-keto-$5\alpha$-androstan-17-one-ethylenedioxy ketal.

$3\alpha$-azido-$5\alpha$-androstane-2,17-dione-17-ethylenedioxy ketal (20.0 g) was dissolved in toluene (400 ml) and silica gel (200 g; Kieselgel 0.05—0.2 mm) was added. The resulting mixture was stirred at room temperature for 24 hours. Ether (200 ml) was now added, the mixture filtered, washed with ether and evaporated under reduced pressure to give a yellow oil. Crystallisation from methanol afforded pure $3\beta$-azido-$5\alpha$-androstane-2,17-dione-17-ethylenedioxy ketal (14.56 g; 72.8%) as prisms, m.p. 139—141°C. $[\alpha]_D$+51.4°; $CHCl_3$; C% 1.062.

d) $3\beta$-azido-$2\beta$-hydroxy-$5\alpha$-androstan-17-one-ethylenedioxy ketal.

$3\beta$-azido-$5\alpha$-androstane-2,17-dione-17-ethylenedioxy ketal (12.0 g) was suspended in methanol (240 ml) and cooled to <10°C in an ice/water bath. Sodium borohydride (3.0 g) was added portionwise to the stirred suspension over 10 minutes and the resulting solution stirred at room temperature for 1 hour. The methanol was evaporated to low volume under reduced pressure and water (200 ml) added. The resultant mixture was extracted with ether, the ether extracts washed with water, dried ($Na_2SO_4$) and evaporated to give an off-white solid. Crystallisation from ether/petrol afforded pure $3\beta$-azido-$2\beta$-hydroxy-$5\alpha$-androstan-17-one-ethylenedioxy ketal (10.0 g; 82.9%) as needles, m.p. 123—125°C. $[\alpha]_D$+0.7°; $CHCl_3$; C% 0.823.

e) $3\beta$-amino-$2\beta$-hydroxy-$5\alpha$-androstan-17-one-ethylenedioxy ketal.

$3\beta$-azido-$2\beta$-hydroxy-$5\alpha$-androstan-17-one-ethylenedioxy ketal (10.0 g) was dissolved in sodium dried ether and added to a stirred suspension of $LiAlH_4$ (2.5 g) in sodium dried ether (50 ml) at <10°C. After the addition the mixture was refluxed 1 hour, cooled in an ice-bath and the excess $LiAlH_4$ destroyed by adding water. The salts formed were filtered through dicalite and the filtrates washed with water, dried ($Na_2SO_4$) and evaporated to give a white solid. The crude product was crystallised from $CH_2Cl_2/Et_2O$ to give pure $3\beta$-amino-$2\beta$-hydroxy-$5\alpha$-androstan-17-one-ethylenedioxy ketal (8.50 g; 91.3%) as prisms, m.p. 200—202°C. $[\alpha]_D$ +7.3°; EtOH; C% 1.83.

f) $3\beta$-amino-$2\beta$-hydroxy compounds.

In a similar way as described in Example I b) — e) the following compounds were prepared from the corresponding $3\alpha$-azido compounds:

$3\beta$-amino-$5\alpha$-androstane-$2\beta$,$17\beta$-diol $17\beta$-acetate,
$3\beta$-amino-$2\beta$-hydroxy-$5\alpha$-pregnan-20-one ethylenedioxy ketal,
$3\beta$-amino-$2\beta$-hydroxy-$5\alpha$-oestran-17-one ethylenedioxy ketal,
$3\beta$-amino-$5\alpha$-oestrane-$2\beta$,$17\beta$-diol $17\beta$-acetate.

Example II

$3\beta$-amino-$2\beta$-hydroxy-$5\alpha$-androstan-17-one

$3\beta$-amino-$2\beta$-hydroxy-$5\alpha$-androstan-17-one-ethylenedioxy ketal (8.42 g) was dissolved in a mixture of glacial acetic acid (168.4 ml) and water (16.8 ml) and heated on a steam bath for 30 minutes. Water was now added and the mixture basified. The precipitated product was filtered, washed base free and dried. Crystallisation from $CH_2Cl_2/Et_2O$ afforded pure $3\beta$-amino-$2\beta$-hydroxy-$5\alpha$-androstan-17-one (6.42 g; 87.2%) as an amorphous solid m.p. >300°C (decomposes), $[\alpha]_D$+105.5°; $CHCl_3$; C% 1.056.

In a similar way the ketals of Example I f) were hydrolyzed to the corresponding 17-ketones.

Example III

1 g of $3\beta$-amino-$2\beta$-hydroxy-$5\alpha$-androstan-17-one was converted with hydrochloric acid to the hydrochloride, which was recrystallised from $EtOH/Et_2O$ to give pure $3\beta$-amino-$2\beta$-hydroxy-$5\alpha$-androstan-17-one hydrochloride as an amorphous solid, m.p. >300°C (decomposes). $[\alpha]_D$+102.2; MeOH; C% 0.629.

In a similar way the following acid addition salts were prepared:

$3\beta$-amino-$2\beta$-hydroxy-$5\alpha$-pregnan-20-one hydrochloride,

3β-amino-2β-hydroxy-5α-oestran-17-one hydrobromide,
3β-amino-5α-androstane-2β,17β-diol 17β-acetate hydrochloride.

Example IV

3β-formamido-2β-hydroxy-5α-androstan-17-one

3β-amino-2β-hydroxy-5α-androstan-17-one (5.5 g) was dissolved in formic acid (55 ml) and heated at 100°C for 6 hours. Water was now added and the mixture basified. The mixture was extracted with $CH_2Cl_2$ and the extracts washed with water, dried ($Na_2SO_4$) and evaporated to give a white solid. Crystallisation from $CH_2Cl_2/Et_2O$ afforded pure 3β-formamido-2β-hydroxy-5α-androstan-17-one (5.22 g, 87%) as prisms, m.p. 283—286°C $[\alpha]_D$+101.2°; $CHCl_3$; C% 1.235.

b) 3β-formamido-2β-hydroxy-5α-androstan-17-one-ethylenedioxy ketal

3β-formamido-2β-hydroxy-5α-androstan-17-one (5.0 g) was suspended in ethylene glycol (20 ml), triethylorthoformate (30 ml) an p-toluene sulphonic acid (0.3 g). The solution obtained on heating was refluxed under nitrogen for 2 hours, poured into $Na_2CO_3$ solution and the precipitated solid filtered, washed with water and dried. The above reaction sequence was repeated in order to get complete conversion to the ketal. Crystallisation of the crude product from $CH_2Cl_2/Et_2O$ afforded pure 3β-formamido-2β-hydroxy-5α-androstan-17-one ethylenedioxy ketal (2.13 g; 37.6%) as prisms, m.p. 252—255°C.

c) 2β-hydroxy-3β-methylamino-5α-androstan-17-one-ethylenedioxy ketal.

3β-formamido-2β-hydroxy-5α-androstan-17-one-ethylenedioxy ketal (2.05 g) was dissolved in sodium dried tetrahydrofuran (40 ml) and added dropwise to a suspension of $LiAlH_4$ (1.02 g) in tetrahydrofuran (40 ml) at <10°C. After the addition was complete the mixture was refluxed 15 minutes, cooled to <10°C and the excess $LiAlH_4$ destroyed by adding water. The salts formed were filtered through Dicalite (registered trademark) and the filtrates washed with water, dried ($Na_2SO_4$) and evaporated to give a clear oil. The crude product was crystallised from $CH_2Cl_2/Et_2O$ to give pure 2β-hydroxy-3β-methylamino-5α-androstan-17-one-ethylenedioxy ketal. (1.72 g; 87.3%) as prisms, m.p. 211—215°C. $[\alpha]_D$ 0°; $CHCl_3$; C% 1.008.

d) 2β-hydroxy-3β-methylamino-5α-androstan-17-one

2β-hydroxy-3β-methylamino-5α-androstan-17-one-ethylenedioxy ketal (1.6 g) was dissolved in a mixture of glacial acetic acid (32 ml) and water (3.2 ml) and heated on a steam bath for 30 minutes. Water was now added and the reaction basified. The mixture was extracted with $CH_2Cl_2$ and the extracts washed with water, dried ($Na_2SO_4$) and evaporated to give a white solid. Crystallisation from $CH_2Cl_2/Et_2O$ afforded pure 2β-hydroxy-3β-methylamino-5α-androstan-17-one (1.11 g; 78.7%).

e) 1 g of 2β-hydroxy-3β-methylamino-5α-androstan-17-one was converted to the hydrochloride, which was recrystallised from $EtOH/Et_2O$ to give pure 2β-hydroxy-3β-methylamino-5α-androstan-17-one hydrochloride as prisms, m.p. >300°C (decomposes). $[\alpha]_D$+103.4°; MeOH; C% 0.544.

f) In a similar manner as described above the following compounds were prepared from the corresponding 3β-amino compounds:
2β-hydroxy-3β-methylamino-5α-oestran-17-one,
2β-hydroxy-3β-methylamino-5α-pregnan-20-one,
3β-methylamino-5α-androstan-2β,17β-diol,
which were then converted into their hydrochlorides.

**Claims for the Contracting States: BE, CH, DE, FR, GB, IT, LU, NL, SE**

1. Steroids of the androstane, oestrane and pregnane series having the formula I:

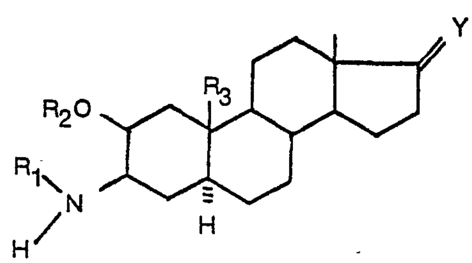

I

and pharmaceutically acceptable non-toxic acid addition salts of these compounds, wherein:

(a) $R_1$ is H, alkyl of one to six carbons (preferably lower alkyl, such as methyl), or acyl of one to eighteen carbons;

(b) $R_2$ is H or acyl of one to eighteen carbons;

(c) $R_3$ is H or methyl, preferably methyl;

(d) Y is selected from the group consisting of O, acetalized O, H($\beta OR_4$), $R_5$($\beta OR_4$), H($\beta COCH_3$) and H($\beta CHOR_4CH_3$);

(e) $R_4$ is H or acyl of one to eighteen carbons; and

(f) $R_5$ is a saturated or unsaturated alkyl group of one to four carbons.

2. Steroids of the androstane series having the formula II:

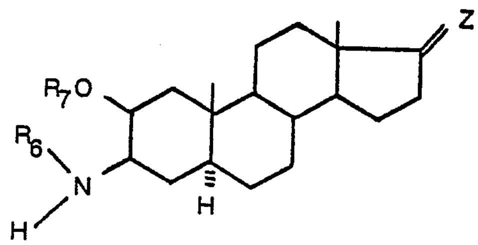

II

and pharmaceutically acceptable acid addition salts and N-acetyl derivatives of these compounds, wherein:

(a) $R_6 = H$ or methyl;

(b) $R_7 = H$ or alkanoyl of 1—4 C-atoms, and

(c) $Z = O$, acetalized O or H($\beta OR_8$), wherein $R_8 = H$ or alkanoyl of 1—4 C-atoms.

3. Steroids of the androstane series having the formula III:

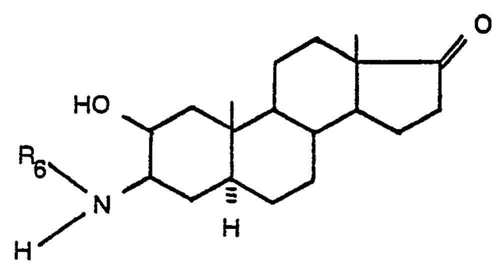

III

wherein $R_6 = H$ or methyl; and the HCl-salt thereof.

4. A pharmaceutical composition having anti-arrhythmic properties, comprising a pharmaceutically effective amount of one or more of the compounds of claim 1, 2 or 3 in admixture with a usual pharmaceutical carrier.

**Claims for the Contracting State: AT**

1. Process for preparing steroids of the androstane, oestrane and pregnane series having the formula I

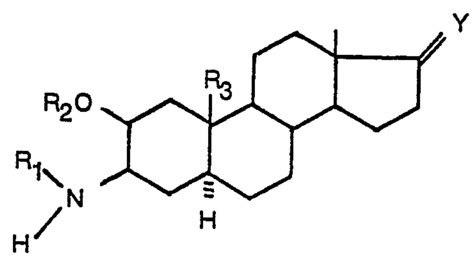

I

and pharmaceutically acceptable non-toxic acid addition salts of these compounds, in which formula

(a) $R_1$ is H, alkyl of one to six carbons (preferably lower alkyl, such as methyl), or acyl of one to eighteen carbons;

(b) $R_2$ is H or acyl of one to eighteen carbons;

(c) $R_3$ is H or methyl, preferably methyl;

(d) Y is selected from the group consisting of O, acetalized O, H($\beta OR_4$), $R_5$($\beta OR_4$), H($\beta COCH_3$) and H($\beta CHOR_4CH_3$);

(e) $R_4$ is H or acyl of one to eighteen carbons; and

(f) $R_5$ is a saturated or unsaturated alkyl group of one to four carbons,

comprising the following steps:

a) oxidation of a corresponding 3α-azido-2β-hydroxy-steroid to give the 3α-azido-2-oxo compound;

b) epimerization of the 3α-azido-2-oxo compound to the 3β-azido-2-oxo compound;

c) reduction of the 3β-azido-2-oxo compound with a complex metal hydride to give the 3β-amino-2β-hydroxy compound;

d) if required, N-formylation and reduction or N-alkylation of the 3β-amino-2β-hydroxy compound to obtain the 3β-alkylamino-2β-hydroxy compound;

e) if required, oxidation of a hydroxy group in position 17 or position 20 (if present) to a corresponding oxo group;

f) if required, reduction of an oxo group in position 17 or position 20 (if present) to a corresponding hydroxy group;

g) if required, acylation of a hydroxy or an amino group to give the corresponding acyloxy or N-acyl group;

h) if required, formation of acid addition salts of the amino compounds by reaction with an acid; and

i) if required, temporary protection of oxo groups and/or hydroxy groups in any of the above reactions by reversible acetal formation and/or acyloxy formation.

2. The process of claim 1 comprising the preparation of steroids of the androstane series having the formula II

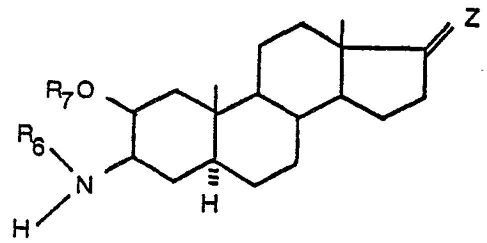

II

and pharmaceutically acceptable acid addition salts and N-acetyl derivatives of these compounds, wherein:

(a) $R_6 = H$ or methyl;

(b) $R_7 = H$ or alkanoyl of 1—4 C-atoms, and

(c) $Z = O$, acetalized O or $H(\beta OR_8)$, wherein $R_8 = H$ or alkanoyl or 1—4 C-atoms.

3. The process of claim 1 comprising the preparation of steroids of the androstane series having the formula III

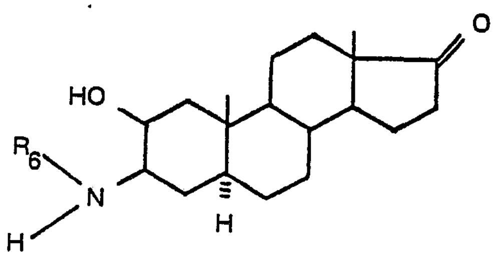

III

wherein $R_6 = H$ or methyl; and the HCl-salt thereof.

**Patentansprüche für die Vertragsstaaten: BE, CH, DE, FR, GB, IT, LU, NL, SE**

1. Steroide der Androstan-, Oestran- und Pregnanreihe der Formel I:

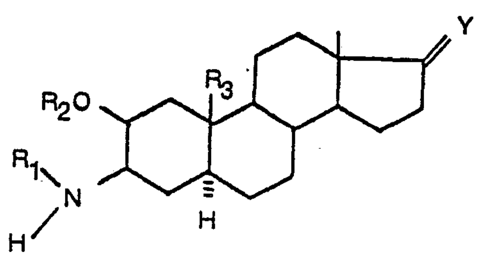

(I)

und pharmazeutisch annehmbare, nicht-toxische Säureadditionssalze dieser Verbindungen, in welcher:

(a) $R_1$ H, Alkyl mit 1 bis 6 Kohlenstoffatomen (vorzugsweise Niederalkyl, wie Methyl) oder Acyl mit 1 bis 18 Kohlenstoffatomen;

(b) $R_2$ H oder Acyl mit 1 bis 18 Kohlenstoffatomen ist;

(c) $R_3$ H oder Methyl, vorzugsweise Methyl ist;

(d) Y ausgewählt ist aus der Gruppe bestehend aus O, acetalisiertem O, H($\beta OR_4$), $R_5$($\beta OR_4$), H($\beta COCH_3$) und H($\beta CHOR_4CH_3$);

(e) $R_4$ oder Acyl mit 1 bis 18 Kohlenstoffatomen ist, und

(f) $R_5$ eine gesättigte oder ungesättigte Alkylgruppe mit 1 bis 4 Kohlenstoffatomen ist.

2. Steroide der Androstanreihe der Formel II:

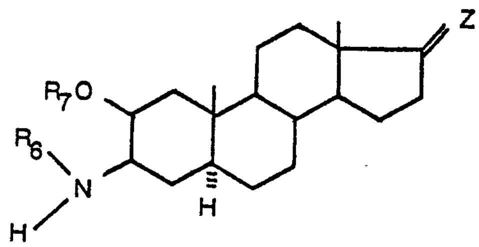

(II)

und pharmazeutisch annehmbare Säureadditionssalze und N-Acetylderivate dieser Verbindungen, worin

(a) $R_6$ H oder Methyl;

(b) $R_7$ H oder Alkanoyl mit 1 bis 4 Kohlenstoffatomen, und

(c) Z O, acetylsiertes O oder H($\beta OR_8$), worin $R_8$ = H oder Alkanoly mit 1 bis 4 Kohlenstomen bedeuten.

3. Steroide der Androstanreihe der Formel III:

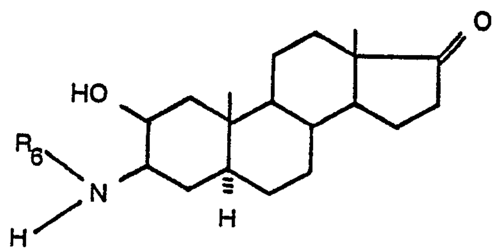

(III)

in welcher $R_6$ = H oder Methyl; und HCl-Salz davon dedeuten.

4. Pharmazeutisches Präparat mit antiarrythmischen Eigenschaften, enthaltend eine pharmazeutische wirksame Menge von einer oder mehreren der Verbindungen von Anspruch 1, 2 oder 3 im Gemisch mit einem üblichen pharmazeutischen Träger.

**Patentansprüche für den Vertragsstaat: AT**

1. Verfahren zur Herstellung von Steroiden der Androstan-, Oestran und Pregnanreihe der Formel I

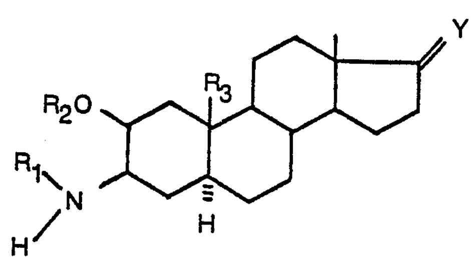

(I)

und pharmazeutisch annehmbaren nicht-toxischen Säureadditionssalzen dieser Verbindungen, in welcher Formel:

(a) $R_1$ H, Alkyl mit 1 bis 6 Kohlenstoffatomen (vorzugsweise Niederalkyl, wie Methyl) oder Acyl mit 1 bis 18 Kohlenstoffatomen ist;

(b) $R_2$ H oder Acyl mit 1 bis 18 Kohlenstoffatomen ist;

(c) $R_3$ H oder Methyl, vorzugsweise Methyl ist;

(d) Y ausgewählt ist aus der Gruppe bestehend aus O, acetalisiertem O, H($\beta OR_4$), $R_5$($\beta OR_4$), H($\beta COCH_3$) und H($\beta CHOR_4CH_3$);

(e) $R_4$ H oder Acyl mit 1 bis 18 Kohlenstoffatomen und

(f) $R_5$ eine gesättigte oder ungesättigte Alkylgruppe mit 1 bis 4 Kohlenstoffatomen ist, umfassend die folgenden Stufen:

a) Oxidation eines entsprechenden 3α-Azido-2β-hydroxysteroides, um die 3α-Azido-2-oxoverbindung zu ergeben;

b) Epimerisierung der 3α-Azido-2-oxoverbindung zur 3α-Azido-2-oxoverbindung;

c) Reduktion der 3β-Azido-2-oxoverbindung mit einem komplexen Metallhydrid, um die 3β-Amino-2β-hydroxyverbindung zu ergeben;

d) falls erforderlich, N-Formulyierung und Reduktion oder N-Alkylierung der 3β-Amino-2β-hydroxy-verbindung, um die 3β-Alkylamino-2β-hydroxyverbindung zu erhalten;

e) falls erforderlich, Oxidation einer Hydroxygruppe in Stellung 17 oder Stellung 20 (falls vorhanden) zu einer entsprechenden Oxogruppe;

f) falls erforderlich, Reduktion einer Oxogruppe in Stellung 17 oder Stellung 20 (falls vorhanden) zu einer entsprechenden Hydroxygruppe;

g) falls erforderlich, Acylierung einer Hydroxy- oder einer Aminogruppe, um die entsprechende Acyloxy- oder N-Acylgruppe zu ergeben;

h) falls erforderlich, Bildung von Säureadditionssalzen der Aminoverbindungen durch Reaktion mit einer Säure; und

i) falls erforderlich, temporärer Schutz von Oxogruppen und/oder Hydroxygruppen in jeder der obigen Reaktionen durch reversible Acetalbildung und/oder Acyloxybildung.

2. Verfahren nach Patentanspruch 1, umfassend die Herstellung von Steroiden der Androstanserie der Formel II:

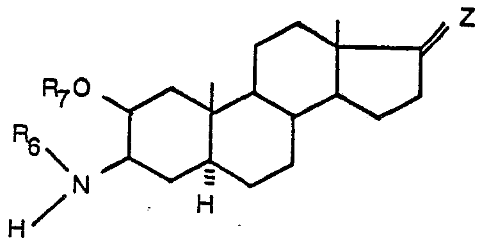

(II)

und pharmazeutisch annehmbaren Säureadditionssalzen und N-Acetylderivaten dieser Verbindung, worin:

(a) $R_6 = H$ oder Methyl;

(b) $R_7 = H$ oder Alkanoyl mit 1 bis 4 Kohlenstoffatomen und

(c) $Z = O$, acetalisiertes O oder $H(\beta OR_8)$, worin $R_8 = H$ oder Alkanoyl mit 1 bis 4 Kohlenstoffatomen bedeuten.

3. Verfahren nach Patentanspruch 1, umfassend die Herstellung von Steroiden der Androstanserie der Formel III:

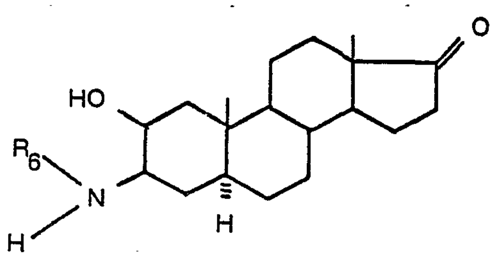

(III)

in welcher $R_6 = H$ oder Methyl, und der HCl-Salze davon, bedeuten.

**Revendications pour les Etats Contractants: BE, CH, DE, FR, GB, IT, LU, NL, SE.**

1. Stéroïdes des séries de l'androstane, de l'oestrane et du pregnane, répondant à la formule I:

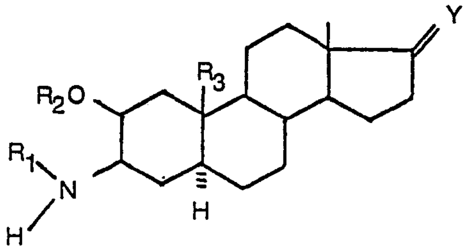

I

et les sels d'addition d'acides non toxiques acceptables en pharmacie de ces composés, où:

(a) $R_1$ représente H ou un radical alkyle comportant 1 à 6 atomes de carbone (de préférence un radical alkyle inférieur tel que méthyle) ou acyle comportant 1 à 18 atomes de carbone;

(b) $R_2$ représente H ou un radical acyle comportant 1 à 18 atomes de carbone;

(c) $R_3$ représente H ou un radical méthyle, de préférence un radical méthyle;

(d) Y est choisi parmi le groupe constitué par O, O acétalisé, H($\beta OR_4$), $R_5$($\beta OR_4$), H($\beta COCH_3$), et H($\beta CHOR_4CH_3$);

(e) $R_4$ représente H ou un radical acyle comportant 1 à 18 atomes de carbone; et

(f) $R_5$ représente un radical alkyle saturé ou insaturé comportant 1 à 4 atomes de carbone.

2. Stéroïdes de la série de l'androstane, répondant à la formule II:

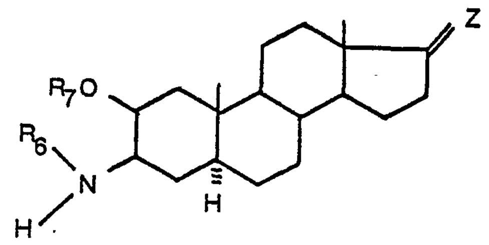

II

et les sels d'addition d'acides acceptables en pharmacie et les dérivés de type N-acétyle de ces composés où:

(a) $R_6$ représente H ou un radical méthyle;

(b) $R_7$ représente H ou un radical alcanoyle comportant 1 à 4 atomes de carbone, et

(c) Z représente O, O acétalisé ou H($\beta OR_8$) où $R_8$ représente H ou un radical alcanoyle comportant 1 à 4 atomes de carbone.

3. Stéroïdes de la série de l'androstane répondant à la formule III

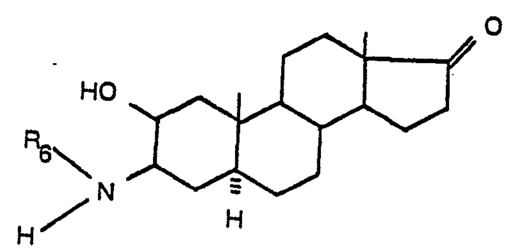

III

où $R^6$ représente H ou un radical méthyle, et leurs chlorhydrates.

4. Composition pharmaceutique ayant des propriétés antiarythmiques comprenant une quantité efficace au point de vue pharmaceutique d'un ou plusieurs des composés des revendications 1, 2 ou 3 en mélange avec un véhicule pharmaceutique habituel.

**Revendications pour l'Etat Contractant: AT**

1. Procédé de préparation de stéroïdes des séries de l'androstane, de l'oestrane et du pregnane, répondant à la formule I:

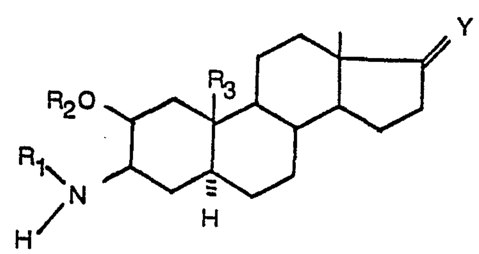

I

et les sels d'addition d'acides non toxiques acceptables en pharmacie de ces composés, où:

(a) $R_1$ représente H ou un radical alkyle comportant 1 à 6 atomes de carbone (de préférence un radical alkyle inférieur tel que méthyle) ou acyle comportant 1 à 18 atomes de carbone;

(b) $R_2$ représente H ou un radical acyle comportant 1 à 18 atomes de carbone;

(c) $R_3$ représente H ou un radical méthyle, de préférence un radical méthyle;

(d) Y est choisi parmi le groupe constitué par O, O acétalisé, H($\beta OR_4$), $R_5$($\beta OR_4$), H($\beta COCH_3$) et H($\beta CHOR_4CH_3$);

(e) $R_4$ représente H ou un radical acyle comportant 1 à 18 atomes de carbone; et

(f) $R_5$ représente un radical alkyle saturé ou insaturé comportant 1 à 4 atomes de carbone comprenant les stades suivants:

a) oxydation du 3α-azido-2β-hydroxy-stéroïde pour donner le composé 3α-azido-2-oxo;

b) épimérisation du composé 3α-azido-2-oxo en le composé 3β-azido-2-oxo;

c) réduction du composé 3β-azido-2-oxo avec un hydrure de métal complexe pour donner le composé 3β-amino-2β-hydroxy;

d) si désiré, N-formylation et réduction ou N-alkylation du composé 3β-amino-2β-hydroxy pour obtenir le composé 3β-alkylamino-2β-hydroxy;

e) si désiré, oxydation d'un radical hydroxy en position 17 ou position 20 (s'il y en a une) en groupe oxo correspondant;

f) si désiré, réduction d'un groupe oxo en position 17-ou position 20 (s'il y en a une) en groupe hydroxy correspondant;

g) si désiré, acylation d'un groupe hydroxy ou amino pour donner le groupe acyloxy ou N-acyle correspondant;

h) si désiré, formation de sels d'addition d'acides des composés amino par réaction avec un acide; et

i) si désiré, protection temporaire de groupes oxo et/ou groupes hydroxy dans l'une quelconque des réactions ci-dessus par formation réversible d'acétal et/out formation d'acyloxy.

2. Le procédé selon la revendication 1, comprenant la préparation de stéroïdes de la série de l'androstane répondant à la formule II:

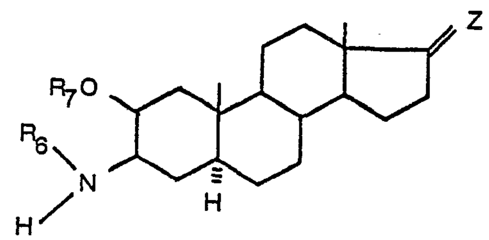

II

et les sels d'addition d'acides acceptables en pharmacie et les dérivés de type N-acétyle de ces composés où:

(a) $R_6$ représente H ou un radical méthyle;

(b) $R_7$ représente H ou un radical alcanoyle comportant 1 à 4 atomes de carbone, et

(c) Z représente O, O acétalisé ou H(β$OR_8$) où $R_8$ représente H ou un radical alcanoyle comportant 1 à 4 atomes de carbone.

3. Le procédé de la revendication 1, comprenant la préparation de stéroïdes de la série de l'androstane répondant à la formule III:

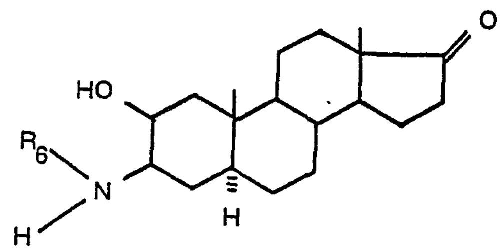

III

où $R_6$ représente H ou un radical méthyle, et leurs chlorhydrates.